# EUROPEAN PATENT APPLICATION

(11) **EP 1 908 456 A1**
(43) Date of publication of application: **09.04.2008**
(21) Application number: 06121723.8
(22) Date of filing: 04.10.2006
(51) Int. Cl.: A61K 8/92, A61Q 5/06

(54) **Non-fluid hair treatment product comprising hair fixative absorbed on waxy carrier**

(71) Applicant: Wella Aktiengesellschaft, 64274 Darmstadt (DE)
(72) Inventor: Walter, Andrea, 64572, Buettelborn (DE); Birkel, Susanne, 64285, Darmstadt (DE); Moenks, Monika, 3185, Schmitten (CH)

(57) **Abstract**

The present invention relates to non-fluid hair treatment products comprising a fluid composition which is absorbed on a waxy carrier, wherein the fluid composition contains at least one hair fixing agent, e.g. a hair fixing polymer. The products can be made by pulverizing a fluid hair styling composition on a non-fluid, waxy carrier, especially by first dissolving a gas in a fluid hair styling composition at high pressure, then expanding the liquid/gas solution, wherein a solid, waxy carrier is added in solid form before, during or shortly after the expansion. The product can be in the form of snow-like flakes which are easily melting in the hand and can be easily distributed on human hair.

## Description

### FIELD OF THE INVENTION

The present invention relates to non-fluid hair treatment products comprising a fluid composition which is absorbed on a waxy carrier, wherein the fluid composition contains at least one hair fixing agent, e.g. a hair fixing polymer. The products can be made by pulverizing a fluid hair styling composition on a non-fluid, waxy carrier, especially by first dissolving a gas in a fluid hair styling composition at high pressure, then expanding the liquid/gas solution, wherein a solid, waxy carrier is added in solid form before, during or shortly after the expansion.

### BACKGROUND OF THE INVENTION

Hair styling products are intended for helping to create individual hair styles and for temporarily holding them in place for a period of time. There are two main product categories: "finishing or setting products" and "style-controlling" products. Finishing products are applied after a hair style has been created to hold the hair style in place by binding the hairs together. Typical finishing products are hair sprays. Style controlling products are applied to the hair before or during the process of creating the desired hair style in order to facilitate this process. They are also called "styling aids" or "modeling products". Among typical styling aid products are styling lotions, styling gels and styling waxes. Gels and lotions are typically hydrophilic fluid compositions of polymers dissolved in aqueous or aqueous-ethanolic solvents which may contain thickeners and gel formers, whereas styling waxes are typically hydrophobic solid compositions containing oily and waxy compounds, but they also can be based on waxy polyethylene glycols. Styling waxes are products that structure the hairstyle and make the hair shine by coating the hair strands with an oily or waxy film. They are essentially anhydrous bases (i.e. containing below about 1% of water) composed either exclusively of fatty substances or of emulsions or of solid polyethylene glycols. They include waxes behaving as thickeners and hardeners. Hydrophobic waxes can be transported in hydrophobic carriers such as mineral oil, hydrocarbons or silicones. Given the problems with removing when washing the hair, the styling wax products must retain a solid texture that limits the amount used when applying to the hair. Fluid styling aids such as lotions and gels provide better distributability and stronger hold compared to solid styling wax products whereas the wax products are better in providing more shine and controllability. Furthermore, the texture and feeling to the touch of both, conventional gels and conventional hair wax products is not totally agreeable. It is therefore one object of the invention to provide combined benefits of hydrophilic fluid styling products and solid styling products in a single product, i.e. without the need of applying two different products to the hair consecutively. It is another object of the invention to provide a solid hair treatment product with easy distributability, improved feeling to the touch and a unique texture, similar to snow flakes melting in the hand when rubbing.

### SUMMARY OF THE INVENTION

The present invention is directed to a non-fluid hair treatment product comprising a fluid composition which is absorbed on a waxy carrier, wherein the fluid composition contains at least one hair fixing agent. Preferred is a non-fluid hair treatment product that is made by pulverizing a fluid hair styling composition (A) on a non-fluid, waxy carrier (B), wherein composition (A) contains at least one hair fixing polymer dissolved or dispersed in at least one solvent which is liquid at room temperature (25 °C), and wherein said waxy carrier (B) is solid at room temperature (25 °C) and comprises at least one waxy substance. The non-fluid hair treatment product can be made by first dissolving a gas in a fluid hair styling composition at high pressure, then expanding the liquid/gas solution, wherein a waxy carrier is added in solid form either before, or during or shortly after the expansion.

The present invention is further directed to methods of hair treatment using the non-fluid hair treatment product. These and other features, aspects, and advantages of the present invention will become evident to those skilled in the art from a reading of the present disclosure.

### DETAILED DESCRIPTION OF THE INVENTION

The hair treatment products of the present invention are of a non-fluid consistency. They are preferably solid or semi-solid and comprise a fluid hair styling composition containing at least one hair fixing agent and wherein this fluid hair styling composition is absorbed on a waxy carrier.

Each of the components, as well as preferred or optional components and the method of making, is described in detail hereinafter. All percentages, parts and ratios are based upon the total weight of the compositions of the present invention, unless otherwise specified. All such weights as they pertain to listed ingredients are based on the active level and therefore do not include solvents or by-products that may be included in commercially available materials, unless otherwise specified. All molecular weights as used herein are weight average molecular weights expressed as grams/mole, unless otherwise specified.

Herein, "comprising" means that other steps and other ingredients which do not affect the end result can be added. This term encompasses the terms "consisting of" and "consisting essentially of". The compositions and methods of the present invention can comprise, consist of, and consist essentially of the essential elements and limitations of the invention described herein, as well as any of the additional or optional ingredients, components, steps, or limitations described herein.

The term "hydrophobic" as used herein, mean substances which are substantially water insoluble, but soluble in an oil phase, with the solubility in the oil phase being higher than that in water or in an aqueous phase. The term "hydrophilic" as used herein, means substances which are substantially water soluble and oil insoluble with the solubility in water or the aqueous phase being higher than that in the oil phase. The term "oil phase" as used herein, means a liquid phase which is separated from water or the aqueous phase. The term "aqueous phase" as used herein, means a liquid phase which comprises water and can additionally comprise hydrophilic co-solvents and water soluble substances.

The term "non-fluid" as used herein, means compositions that do not flow at ambient conditions, for example they do not run off a sheat of glass tilted at an angle of 45° and at a temperature of 25 °C. Non-fluid compositions are preferably solid, semi-solid, paste-like or cream-like. Most preferred they are solid with a melting point above 25°C. The term "fluid" as used herein, means either compositions that are non-viscous, i.e. have viscosities similar to water such as aqueous or aqueous-ethanolic lotions or compositions that are thickened with a gelifier up to viscosities of up to e.g. 100000 mPa s, as long as they are at least squeezable from tube packagings, i.e. flow under increased shear stress. The term "fluid" also comprises higher viscous consistencies such as e.g. soft creams, semi-solids or semi-solid waxes which are flowable at least under higher pressure of e.g. from 5 to 800 bar. All viscosities mentioned herein are measured as dynamic viscosity with a Haake VT-550 Rheometer, measurement body SV-DIN at a temperature of 25° C and at a shear rate of 50 s⁻¹, unless otherwise cited. The terms "wax" and "waxy" as used herein, means a composition or substance that has the haptic and texture properties typical for a wax-like product such as paraffin wax, e.g. is non-fluid and plastic at 20°C and softens or becomes fluid under shear or warming and melts over 25°C, preferably over 40°C, without decomposition.

Hair fixing or hair styling agents are compounds which impart hair-holding or style-retention properties to hair, e.g. increase the curl retention of human hair when applied as 0,01 to 5% by weight aqueous, alcoholic or aqueous-alcoholic solution or dispersion when compared with a treatment with water only. In particular, hair fixing polymers are those polymeric compounds listed in the International Cosmetic Ingredient Dictionary and Handbook, 11^{th} edition 2006 with the function "Hair Fixatives".

All cited references are incorporated herein by reference in their entireties. Citation of any reference is not an admission regarding any determination as to its availability as prior art to the claimed invention.

### Fluid composition

The fluid composition that is absorbed on the waxy carrier contains at least one hair fixing agent which is dissolved or dispersed in a suitable cosmetically acceptable solvent. The fluid composition may be thickened or gelled by suitable thickeners or gelling agents. Preferred hair fixing agents are hair fixing polymers.

### Hair fixing polymers

The amount of hair fixing polymer is preferably from 0,01 to 30, more preferably from 0,1 to 15, and most preferred from 0,5 to 10% by weight based on the fluid composition. The hair fixing polymer can be nonionic, anionic, cationic, amphoteric or zwitterionic, preferably it is nonionic or anionic. The hair fixing polymer can be synthetic or natural. The term "natural polymer" also comprises chemically modified polymers of natural origin. Preferred are polymers which are soluble in an aqueous or aqueous-alcoholic solvent.

Suitable synthetic, non-ionic hair fixing polymers are for example:
homo- oder copolymers of at least one monomer selected from vinyl pyrrolidone; vinyl caprolactam; vinyl ester e.g. vinyl acetate, vinyl alcohol, acrylamide, methacrylamide, alkyl- and dialkyl acrylamide, alkyl- und dialkyl methacrylamide, dialkylaminoalkyl methacrylamide, dialkylaminoalkyl acrylamide, alkylacrylate, alkylmethacrylate, propyleneglycol oder ethylenglykol, wherein preferred alkyl groups of these monomers are C1- to C7-alkyl groups, more preferred C1- to C3-alkyl groups. Suitable are e.g. homopolymers of vinyl caprolactam, homopolymers of vinyl pyrrolidone, homopolymers of N-vinyl formamide. Suitable hair fixing polymers are also copolymers of vinyl pyrrolidone and vinyl acetate; terpolymers of vinyl pyrrolidone, vinyl acetate and vinyl propionate; terpolymers of vinyl pyrrolidone, vinyl caprolactam and dialkylaminoalkyl (meth)acrylate; terpolymers of vinyl pyrrolidone, vinyl caprolactam and dialkylaminoalkyl (meth)acrylamide; polyacrylamide; polyvinyl alcohol; and hair fixing polyethylen glycol/polypropylen glycol copolymers. Preferred are nonionic vinyl lactam homo- or copolymers. Suitable vinyl lactams are e.g. vinyl caprolactam and vinylpyrrolidone. Especially preferred are polyvinyl pyrrolidone, polyvinyl caprolactam and polyvinyl pyrrolidone/vinyl acetate copolymers which are marketed e.g as Luviskol® VA 37 und Luviskol® VA 64.

Suitable synthetic, anionic hair fixing polymers can be synthetic or natural homo- or copolymers from monomeric units with acid groups . The monomers with acid groups can be copolymerised with monomers without acid groups. Preferred acid groups are -COOH, - SO₃H, -OSO₃H, -OPO₂H und -OPO₃H₂, carboxylic acid being most preferred. The acid groups can be unneutralised, partially neutralised or completely neutralised. Preferred is a degree of the anionic, neutralised form of from 50 to 100%. Suitable monomers are ethylenically unsaturated, radically polymerisable compounds carrying at least one acid group, e.g. styrene sulfonic acid, 2-acrylamide-2-methylpropane sulfonic acid or carboxyvinyl monomers like acrylic acid, methacrylic acid, crotonic acid, fumaric acid, maleic acid, maleic anhydride and its monoesters or itaconic acid.

Comonomers without acid groups are e.g. acrylamide, methacrylamide, alkyl- and dialkyl acrylamide, alkyl- and dialkyl methacrylamide, alkylacrylate, alkylmethacrylate, vinyl caprolactone, vinyl pyrrolidone, vinyl ester, vinyl alcohol, propylen glycol or ethylen glycol, amine substituted vinyl monomers such as dialkylaminoalkyl acrylate, dialkylaminoalkyl methacrylate, monoalkylaminoalkyl acrylate and monoalkylaminoalkyl methacrylate, wherein preferred alkyl groups are C1- to C7-alkyl groups, especially C1- to C3-alkyl groups.

Suitable anionic hair fixing polymers are in particular copolymers of acrylic or methacrylic acid with monomers selected from acrylic acid esters, methacrylic acid esters, acrylamides, methacrylamides and vinyl pyrrolidone; homopolymers of crotonic acid; copolymers of crotonic acid with monomers selected from vinyl esters, acrylic acid esters, methacrylic acid esters, acrylamides, methacrylamides. A natural anionic hair fixing polymer is shellac. Preferred anionic hair fixing polymers are vinylacetate/crotonic acid copolymer; partially esterified copolymers of vinyl methylether and maleic anhydride; terpolymers of acrylic acid, alkyl acrylate and N-alkyl acrylamide, e.g. acrylic acid/ethyl acrylate/N-t-butyl acrylamide terpolymer; terpolymers of vinyl acetate, crotonic acid and vinyl alkanoate, e.g. vinyl acetate/crotonic acid/vinyl neodecanoate copolymer.

Suitable synthetic, amphoteric hair fixing polymers are polymers with anionic or acidic functional groups as well as cationic or basic functional groups. The acidic or anionic functional groups are those as definied above for the anionic polymers. Cationic or basic functional groups are in particular primary, secondary or tertiary amine groups or quaternary ammonium groups. Preferred examples are copolymers of alkyl acrylamide (especially octyl acrylamide), alkylaminoalkyl methacrylate (especially t-butylaminoethyl methacrylate) and two or more monomers selected from acrylic acid, methacrylic acid and their esters, wherein the alkylgroups have from 1 to 4 C-atoms and at least one of the monomers has an acid group. A marketed product is e.g. Amphomer® oder Amphomer® LV-71 of National Starch. Further examples for hair fixing polymers are copolymers of acrylic acid, methyl acrylate and methacrylamidopropyl trimethylammonium chloride (INCI-name: polyquaternium-47); copolymer of acrylamidopropyl trimethylammonium chloride and acrylates; or copolymers of acrylamide, acrylamidopropyl trimethylammonium chloride, 2-amidopropyl acrylamide sulfonate and dimethylaminopropyl amine (INCI-name: polyquaternium-43). Suitable are also polymers with betaine groups, e.g. copolymers of methacryloyl ethylbetaine and two or more monomers selected from acrylic acid and its alkyl esters (INCI-name Methacryloyl Ethyl Betaine/Acrylates Copolymer).

Suitable cationic hair fixing polymers are polymers with cationic or basic functional groups. Cationic or basic functional groups are in particular primary, secondary or tertiary amine groups or quaternary ammonium groups. The cationic charge density is preferably from 1 to 7 meq/g. The cationic polymers can be homopolymers or copolymers wherein the cationic or basic functional group can be part of the polymeric backbone or can be a pendant group. Monomers with cationic or basic groups can be copolymerised with monomers without cationic or basic group.

Suitable cationic monomers are ethylenically unsaturated radically polymerisable compounds with at least one cationic or basic group, e.g. ammonium substituted vinyl monomers such as trialkyl methacryloxy alkylammonium, trialkyl acryloxy alkyl ammonium, dialkyl diallyl ammonium and quaternary vinyl ammonium monomers with cyclic nitrogen containing groups such as pyridinium, imidazolium or quaternary pyrrolidones, e.g. alkylvinyl imidazolium, alkylvinyl pyridinium. The alkyl groups of these monomers are preferably lower alkyl groups such as C1 to C7 alkyl groups, more preferred C1 to C3 alkyl groups. The cationic monomers can be polymerised with non-cationic comonomers. Non-cationic comonomers are e.g. acrylamide, methacrylamide, alkyl- and dialkyl acrylamide, alkyl- and dialkyl methacrylamide, alkylacrylate, alkylmethacrylate, vinyl caprolactone, vinyl pyrrolidone, vinyl ester such as vinyl acetate, vinyl alcohol, propylen glycol or ethylen glycol, wherein preferred alkyl groups are C1- to C7-alkyl groups, especially C1- to C3-alkyl groups.

Suitable cationic hair fixing polymers are for examples those listed in the International Cosmetic Ingredient Dictionary and Handbook as polyquaternium, e.g. methylvinyl imidazolium chloride/vinyl pyrrolidone copolymer (Polyquaternium-16) or quaternised vinyl pyrrolidone/dimethylaminoethyl methacrylate copolymer (Polyquatemium-11). Preferred synthetic cationic hair fixing polymers are:
poly(dimethyl diallyl ammonium chloride); copolymers of acrylamide and dimethyl diallyl ammonium chloride; quaternary ammonium polymers made by reaction of diethylsulfate and a copolymer of vinyl pyrrolidone and dimethylaminoethyl methacrylate, such as vinyl pyrrolidone/dimethylaminoethyl methacrylate methosulfate copolymer (e.g. GAFQUAT® 755 N, GAFQUAT^{®} 734); quaternary ammonium polymers of methylvinyl imidazolium chloride and vinyl pyrrolidone (e.g. LUVIQUAT^{®} HM 550); Poly-quaternium-35; Polyquaternium-57; polymer of trimethylammoniumethyl methacrylate chloride; terpolymer of dimethyl diallyl ammonium chloride, sodium acrylate and acrylamide (e.g. MERQUAT^{®} Plus 3300); copolymer of vinyl pyrrolidone, dimethylaminopropyl methacrylamide and methacryloyl aminopropyllauryl dimethyl ammonium chloride; terpolymer of vinylpyrrolidone, dimethylaminoethyl methacrylate and vinyl caprolactam (e.g. GAFFIX^{®} VC 713); vinyl pyrrolidone / methacryl amidopropyl trimethylammonium chloride copolymer (e.g. GAFQUAT^{®} HS 100); copolymer of vinyl pyrrolidone and dimethylaminoethyl methacrylate; copolymer of vinyl pyrrolidone, vinyl caprolactam and dimethylaminopropyl acrylamide; poly- or oligoester made of at least one monomer selected from hydroxyacids which are substituted with at least one quaternary ammonium group.

Sutitable cationic polymers derived from natural polymers are for example cationic derivatives of polysaccharides such as cationic derivatives of cellulose, starch or guar. Suitable are also chitosan and chitosan derivatives. Cationic polysaccharides have for example the general formula

G-O-B-N⁺R^{a}R^{b}R^{c} X⁻

Wherein G is an anhydroglucose group such as starch anhydroglucose or cellulose anhydroglucose; B is a divalent bridging group such as alkylen, oxyalkylen, polyoxyalkylen or hydroxyalkylen; R^{a}, R^{b} and R^{c} are independent from one another alkyl, aryl, alkylaryl, arylalkyl, alkoxyalkyl or alkoxyaryl with each up to 18 carbon atoms, wherein the total number of carbon atoms in R^{a}, R^{b} and R^{c} is preferably a maximum of 20; X is a counter ion, such as halogen, acetate, phosphate, nitrate or alkylsulfate, preferably chloride. Cationic cellulose polymers are for example those with the INCI-names Polyquaternium-10 or Polyquaternium-24. A cationic guar derivative is for example that with the INCI-name Guar Hydroxypropyltrimonium Chloride.

Especially preferred cationic hair fixing polymers are chitosan, chitosan salts and chitosan derivatives. Chitosans are totally or partially deacetylated chitines. The molecular weight can be for example from about 20000 to about 5 Millionen g/mol, e.g.. from 30000 to 70000 g/mol for lower molecular weight chitosan. Preferred are high molecular chitosans with a molecular weight above 100000 g/mol, more preferred from 200000 to 700000 g/mol. The degree of deacetylation is preferably from 10 to 99%, more preferred from 60 to 99%. A preferred chitosan salt is chitosonium pyrrolidonecarboxylate, e.g. KYTAMER® PC with a molecular weight of about 200000 to 300000 g/mol and a degree of deacetylation of 70 bis 85%. Chitosan derivatives are for example quaternised chitosans, alkylated chitsoans or hydroxyalkylated chitsoans such as hydroxyethyl-, hydroxypropyl- or hydroxybutyl chitosan. The chitosan or chitosan derivatives are preferably partially or completely neutralised. The degree of neutralisation is preferably at least 50%, more preferred from 70 und 100%, based on the total number of amino groups. In principle, all cosmetic acceptable inorganic or organic acids can be used for neutralisation, such as formic acid, tartaric acid, malic acid, lactic acid, citric acid, pyrrolidone carboxylic acid, glycolic acid, hydrochloric acid etc., pyrrolidone carboxylic acid being especially preferred.

Preferred cationic polymers on a natural basis are: cationic cellulose derivatives made from hydroxyethylcellulose and diallyl dimethyl ammonium chloride; cationic cellulose derivatives made from hydroxyethylcellulose and trimethyl ammonium substituted epoxide; chitosan and ist salts; hydroxyalkyl chitosan and ist salts; alkylhydroxyalkyl chitosan and ist salts; N-hydroxyalkyl chitosan alkylether.

Most preferred hair fixing polymers are polyvinylpyrrolidone (INCI-name PVP; trade names e.g. Luviskol™ K30, K85, K90 available from BASF); copolymers of vinylpyrrolidone and vinylacetate (INCI-name VP/VA copolymer; trade names e.g. Luviskol™ VA37, VA64 available from BASF); copolymers of vinylpyrrolidone, methacrylamide and vinylimidazole (INCI-name VP/Methacrylamide/Vinyl Imidazole Copolymer, trade name Luviset™ Clear available from BASF); copolymers of vinylacetate and crotonic acid (INCI-name VA/Crotonates Copolymer, trade name Luviset™ CA66 available from BASF); copolymers of octylacrylamide, acrylic acid, butylamino methacrylate, methyl methacrylate and hydroxypropyl methacrylate (INCI-name Octylacrylamide/Acrylates/Butylaminoethyl Methacrylate Copolymer, available from National Starch and Chemical Company); copolymer of alkylacrylate, acrylic acid and alkylacrylamide (INCI-name Acrylates/t-Butylacrylamide Copolymer; trade name Ultrahold™ 8 available from BASF).

The fluid composition according to the invention can be aqueous of aqueous-alcoholic. By "aqueous" it is meant that the compositions contain almost only water as solvent, i.e. organic solvents such as C1- to C4 alcohols are not present or they are present only in very minor amounts such as below 2 or below 1% by weight of the fluid composition. Deionized water is preferably used. Water from natural sources containing mineral cations can also be used, depending on the desired characteristic of the product. By "aqueous-alcoholic" it is meant that the compositions contain significant amounts of water as well as significant amounts of alcoholic solvents. Significant amounts are amounts of e.g. at least 5% by weight or more. The level and species of the solvents are selected according to the compatibility with other components, and other desired characteristics of the product. Alcoholic solvents are organic compounds which are liquid at room temperature (25°). The amount of alcohol is preferably 0 to 50% by weight, more preferably from 1 to 25% by weight of the liquid composition. Alcohols can be those conventionally used for cosmetic purposes, e.g. monohydric C1 to C6 alcohols such as ethanol and isopropanol. Ethanol is especially preferred. The water content is preferably from 40 to 95, more preferred from 50 to 90% by weight of the fluid composition. An aqueous-ethanolic carrier can contain for example 5 to 25% by weight ethanol and 60 to 80% by weight water, based on the total composition. The pH is preferably in the range of from 6 to 9, more preferably from 6,5 to 8. Buffers and other pH adjusting agents can be included to achieve or stabilize the desirable pH.

### Polyhydric alcohols

In one embodiment of the invention, the fluid hair styling composition additionally contains at least one polyhydric alcohol for further improving the hair shine. The polyhydric alcohols have at least two alcoholic hydroxyl groups. They have preferably 2 to 6 carbon atoms and 2 to 6 hydroxyl groups such as glycerol, C2- to C4-alkylenglycols, and sorbitol. Especially preferred are glycerol and C2- to C4-alkylenglycols, such as ethylenglycol and propylenglycol. The amount of polyhydric alcohol is preferably from 0,1 to 15, more preferably from 0,5 to 6% by weight based on the fluid composition.

### Gel forming agents

In one embodiment of the invention, the fluid hair styling composition has the form of a gel and additionally contains at least one gel forming agent. The amount of gel forming agents is preferably from 0,05 to 30, more preferably from 0,2 to 20 and most preferably from 0,5 to 10% by weight based on the fluid composition. Suitable gel forming agents are for example one or a mixture of:
- synthetic polymer such as e.g. crosslinked polyacrylates;
- polymers on a natural basis, e.g. based on sclerotium gum; starch; gelatine; cellulose and cellulose derivatives such as carboxymethyl cellulose,hydroxyalkyl cellulose such as hydroxypropylcellulose or hydroxyethylcellulose, methylcelluose or hydroxyproyplmethylcellulose; microcrystalline cellulose; agar-agar; carrageenan, alginates, carouba gum, guar and guar derivatives such as alkylated and hydroxyalkylated guar; karaya gum; xanthan gum; dehydroxanthan; gum arabicum, pektin
- inorganic thickeners, e.g. hectorite, bentonite, metal silicates such as aluminium silicates or magnesium silicates.

In particular, gel forming agents are:
copolymers of at least one first monomer selected from acrylic acid and methacrylic acid and at least one second monomer selected from esters of acrylic acid and ethoxylated fatty alcohols; crosslinked polyacrylic acid; crosslinked copolymers of at least one first monomer selected from acrylic acid and methacrylic acid and at least one second monomer selected from esters of acrylic acid and C10 to C30 alcohols such as those with INCI-name Acrylates/C10-30 Alkyl Acrylate Crosspolymer having tradenames Pemulen™ TR-1, Pemulen™ TR-2, Carbopol™ 1342, Carbopol™ 1382, and Carbopol™ ETD 2020, all available from Noveon, Inc.; copolymers of at least one first monomer selected from acrylic acid and methacrylic acid and at least one second monomer selected from esters of itaconic acid and ethoxylated fatty alcohols; copolymers of at least one first monomer selected from acrylic acid and methacrylic acid and at least one second monomer selected from esters of itaconic acid and ethoxylated C10 to C30 alcohols and at least one third monomer selected from amino C1 to C4-alkylacrylates; copolymers of two or more monomers selected from acrylic acid, methacrylic acid, acrylic acid esters and methacrylic acid esters; copolymers of vinylpyrrolidone and ammonium acryloyl dimethyltaurate; copolymers of ammonium acryloyl dimethyltaurate and at least one monomer selected from esters of methacrylic acid and ethoxylated fatty alcohols; hydroxyethyl cellulose; hydroxylpropyl cellulose; hydroxypropyl guar; glyceryl polyacrylate; glyceryl polymethacrylate; copolymers of styrene and at least one C2, C3 or C4-alkylene; gel forming polyurethanes; hydroxypropyl starch phosphate; polyacrylamide; copolymer of maleic acid anhydride and methylvinylether crosslinked wich decadiene; carob bean gum; guar gum; xanthan; dehydroxanthan; carrageenan; karaya gum; hydrolysed corn starch; copolymers of polyethylenoxide, fatty alcohols und saturated methylene diphenyldiisocyanate (e.g. PEG-150/stearyl alcohol/SMDI copolymer).

Gel forming or hair fixing polymers with acid groups are preferably neutralized up to 50 bis 100%. Non-limiting examples of neutralizing agents include primary or secondary organic amines, or inorganic bases such as ammonia, NaOH, KOH, ammonium hydroxide etc.. Preferred are amino alcohols with 1 to 10 carbon atoms and 1 to 3 hydroxy groups such as aminomethyl propanol (AMP), monethanolamine, diethanol amine, triethanolamine, tetrahydroxypropyl ethylendiamine, diisopropanolamine, tromethamine, and mixtures thereof.

The fluid compositions of the invention can optionally contain a plasticizer for the hair fixing polymers. Any plasticizer suitable for use in hair care products or for topical application to the hair or skin can be used. A wide variety of plasticizers are known in the art. These include acetyl triethylcitrate, triethylcitrate, glycerin, diisobutyl adipate, butyl stearate, phtalates and propylene glycol. Plasticizers are typically used at levels of from about 0.01 % to about 10%, by weight of the fluid composition, preferably from about 0.05% to about 3%, more preferably from about 0.05% to about 1%.

### Rheology

The fluid hair styling composition of the invention can have the form of a non-viscous or slightly viscous lotion, i.e. it contains essentially no gel-former and has a viscosity of less than 100 mPa s. The fluid hair styling composition of the invention can also have the form of a viscous lotion or a gel, especially a fluid gel. Viscous compositions and gel compositions preferably have a viscosity of from at least 250 mPa s, especially from 500 to 100000 mPa s, preferably from 1000 to 50000 mPa s, and most preferably from 2000 to 15000 mPa s.

A preferred embodiment of the fluid composition is a hair styling gel composition having a viscosity of at least 250 mPa s and containing in an aqueous or aqueous-ethanolic solvent
(A) 0.5 to 10 wt.% based on the gel composition of at least one gel forming polymer; and
(B) 0.1 to 15 wt.% based on the gel composition of at least one dissolved or dispersed hair fixing polymer, selected from the group consisting of anionic hair fixing polymers and nonionic hair fixing polymers.

### Waxy carrier

The waxy carrier according to the invention either consists of one or more waxy compounds (hereafter generally called "waxes") or it is a composition containing at least one or more wax in an amount which ensures a waxy texture of the carrier. The exact minimum amount of wax depends on the type of wax used as well as on the type and amounts of optional additives. The compositions of the solid hair wax products according to the invention preferably have a needle penetration number (measurement unit 0.1 mm, test weight 100 g, test duration 5 s, test temperature of 25° C, according to DIN 51 579) of greater than or equal to 10, or 20 and preferably not more than 70. The melting point is preferably 30 °C or higher, most preferred from 40 to 60 °C. The amount of wax is preferably from 1 to 60 wt.%, or from 5 to 60 wt.%, preferably from 10 to 50 wt.%, most preferred from 20 to 50 wt.%, based on the total amount of waxy carrier.

Typical waxes include animal waxes, vegetable waxes, mineral waxes, synthetic waxes, solid paraffin waxes, solid petrolatum (Vaseline®), ozocerite, ceresin, montan wax, Fischer-Tropsch waxes, polyolefin waxes (e.g. polybutene), bees wax, wool wax and its derivatives (e.g. wool wax alcohols solid at room temperature), candelilla wax, olive wax, carnauba wax, japan wax, apple wax, hardened (e.g. hydrogenated) fats, fatty acid esters and fatty acid glycerides with solidification points above 40° C, solid fatty acid triglycerides, solid fatty acids, solid fatty alcohols, polyethylene waxes, silicone waxes, solid di- or oligosaccharide polyesters (e.g. sucrose octaesters with C10 to C26 fatty acids such as sucrose polybehenate) and solid polyethylene glycols.

The waxes preferably have a melting point above 35° C, preferably above 45° C. The needle penetration number (0.1 mm, 100 g, 5 s, 25° C; according to DIN 51 579) is preferably in a range of 2 to 70, especially from 3 to 40. Preferably at least one wax is present in the waxy carrier of the invention, which has a needle penetration point which is less than 40, especially preferably less than 20. Ceresine wax with a needle penetration point of less than 20 or beeswax or their mixtures are especially preferred.

In one embodiment the wax is at least one waxy, solid polyethylene glycol. The preferred amount of waxy polyethylene glycol is at least 20 weight %, e.g. 20 to 90 or 30 to 80 wt.% based on total amount of waxy carrier. The waxy polyethylene glycols have a melting point above 25°C, preferably from 35 to 60 °C or from 45 to 60 °C. The molecular weight can be 950 or more, e.g. from 950 to 30000, preferably from 1800 to 5000 g/mol, or from 2500 to 3500 g/mol. Polyethylene glycols have the general formula H(OCH₂CH₂)ₙOH. Suitable are e.g. those with n = 20 to 220, preferably with n = 40 to 100, or from 50 to 80 and with INCI-names PEG-20, PEG-32, PEG-40, PEG-45, PEG-55, PEG-60, PEG-75, PEG-90 or PEG-100. Most preferred are PEG-60 and PEG-75. Commercially available products usually have a molecular weight distribution. Suitable are e.g. Polyglykol 3000 of Clariant with a molecular weight range of 2700 to 3000 or Polyglykol 4000 with a molecular weight range of 3700 to 4500. In one embodiment the waxy carrier contains 30 to 80 wt.%, based on total amount of carrier, of at least one polyethylene glycol with a molecular weight of from 950 to 30000. Additionally, fluid polyethylene glycols can be contained.

### Oils

In addition to the waxes, the waxy carrier can contain liquid hydrophobic oils. In one embodiment of the invention, the waxy carrier contains at least one hydrophobic oil that is liquid at room temperature. Most preferred are low volatile oils with a boiling point for example of at least 200 °C or at least 250 or 300 °C. The preferred amount of oil is at least 3% by weight, more preferred 5 to 60% or 10 to 50% by weight based on the total amount of waxy carrier and comprises hydrophobic oil that is liquid at 25 °C and optionally dissolved lipophilic materials. The hydrophobic oils and the lipophilic materials of the oil phase can for example be selected from vegetable oils, animal oils, mineral oils, silicone oils, hydrocarbon oils, hydrogenated polyolefins, fatty alcohols with at least 8 carbon atoms including branched alcohols such as guerbet alcohols, oils from fatty acids and polyols (especially triglycerides), oils from fatty acids and monohydric C1- to C30-alcohols (preferred C3- to C22-alcohols) and mixtures of said hydrophobic oils. Non-limiting hydrophobic oils are for example cyclic paraffins, parrafin oils, isoparaffin oils, polydecene, mineral oil, isohexadecane, dodecane, isoeicosane, liquid polydimethylsiloxane, cyclotetrasiloxane, cyclopentasiloxane, phenyltrimethicone, isocetylpalmitate, isopropylmyristate, isopropylpalmitate, isopropylstearate, octylisostearate, octylcocoate, octylpalmitate, octyldodecylmyristate, caprylic/capric triglyceride, butyloctanol, hexyloctanol, butyldecanol, hexyldecanol, octyldodecanol, hexyldecanol, stearylheptanoate, isohexyldecanoate, isodecyloctanoate, dibutyladipate, dicaprylylether, C12-15-alkylbenzoate, hydrogenated polyisobutene, squalane, squalene, native oils such as jojoba oil, olive oil, sunflower oil, soja oil, peanut oil, rape seed oil, sweet almond oil, palm-oil, coconut oil, castor oil, hydrogenated castor oil, wheat germ oil, grape seed oil, safflower oil, evening primrose oil, macedemia nut oil, corn oil, avocado oil, lanolin oils and similar oils. Especially preferred oil compounds are hydrocarbon oil such as mineral oil (e.g. paraffinum liquidum) and branched C8 to C30 alkyl alcohols. Silicone oils include polydimethylsiloxanes, phenylated silicones, polyphenylmethylsiloxanes, phenyltrimethicones, poly-C₁ to C₂₀-alkylsiloxanes and alkylmethylsiloxanes.

### Emulsifiers

Preferred embodiments of the invention additionally include at least one emulsifier either in the fluid composition or in the waxy carrier or in both, in order to improve the washability of the composition from the hair and to further improve the perfomance benefits. The emulsifiers are preferably contained in an amount of from 0.5 to 20% by weight, especially preferably from 3 to 15% by weight. Preferred emulsifiers are selected from the group of non-ionic and anionic surfactants. In a particularly preferred embodiment the emulsifiers have a wax-like consistency and a melting point above 25° C.
Nonionic emulsifiers are for example
- alkoxylated fatty alcohols such as C8- to C30- or preferably C8- to C22-alcohols, alkoxylated fatty acids or alkoxylated fatty acid glycerides such as C 12 to C22-fatty acids, alkoxylated alkylphenols (e.g. alkyl groups with 8 to 15 carbon atoms); typical degrees of ethoxylation being from 2 to 100 or from 4 to 30 and typical degrees of propoxylation being from 1 to 5;
- C8 to C30-, preferably C12- to C22-fatty acid glycerolmono- or diester, ethoxylated with from 1 to 30 mole ethylenoxide;
- Castor oil or hydrogenated castor oil ethoxylated with from 5 to 60 mole ethylenoxide;
- Fatty acid sugar mono- or diester, especially ester of sucrose with one or two C8- to C30 or C12 to C22-fatty acid, INCI: Sucrose Cocoate, Sucrose Dilaurate, Sucrose Distearate, Sucrose Laurate, Sucrose Myristate, Sucrose Oleate, Sucrose Palmitate, Sucrose Ricinoleate, Sucrose Stearate;
- ethoxylated sorbitan esters such as ester of sorbitan with one, two or three C8- to C22-fatty acid and a degree of ethoxylation of from 4 to 20;
- polyglyceryl fatty acid ester, especially of one, two or more C8- to C22-fatty acids with polyglycerol of preferably 2 to 20 glycerol units;
- alkylglucoside, alkyloligoglucoside or alkylpolyglucoside with C8- to C22-alkyl groups, e.g. Decyl Glucoside oder Lauryl Glucoside.

Anionic surfactants are for example alkyl carboxylic acids, alkyl ethersulfates, alkylsulfates, sulfosuccinates, fatty acid isethienates, phosphoric acid alkyl ester, ethoxylated phosphoric acid alkyl ester such as mono- di- or triesters of phosphoric acid with C8- to C22-fatty alcohols ethoxylated with 2 to 30 mol ethylenoxide, acylaminoacids, said acyl groups having preferably 8 to 30 carbon atoms. Preferred emulsifiers are triesters of phosphoric acid with ethoxylated fatty alcohols such as for example the triester of phosphoric acid with cetyl and stearyl alcohol ethoxylated with 4 mol of ethylenoxide (INCI: Triceteareth-4 Phosphate).

According to another embodiment of the invention, the hair treatment products according to the invention are coloured products containing at least one colourant or colour additive. Preferred product colours are oil soluble organic dyes. But insoluble pigments or water soluble or alcohol soluble dyes can also be used. The coloured products preferably contain essentially no or only minor amounts (such as for example less than about 5%, preferably less than about 1% by weight) of non-white waxes such as carnauba wax which are coloured by themselfes. The amount of colourants can vary for example from 0,0001 to 5% by weight. Colourants and colour additives can for example be selected from those listed in the "International Cosmetic Ingredient Dictionary and Handbook", 10^{th} edition, 2004, volume 3, section 3 under the function "colorants".

### Optional ingredients

The products according to the invention can also contain conventional cosmetic additives usually used in hair treatment compositions in addition to the above-mentioned ingredients, e.g. fragrances and perfume oils in an amount of up to 2% by weight, preferably from 0.01 to 1% by weight; preservatives such as for example parabenes, phenoxetol, iodopropynyl carbamate, parahydroxybenzoic acid ester, benzoic acid, salicylic acid, sorbic acid, mandelic acid, polyhexamethylene biguanidine hydrochloride or isothiazoline based compounds in an amount of for example up to 2% by weight, preferably 0.01 to 1% weight; buffer substances, such as sodium citrate or sodium phosphate, in an amount of 0.1 to 1% by weight; hair care substances, such as e.g. moisturizer, betaine, panthenol, plant extracts, vegetable extracts, protein hydrolyzates and silk hydrolyzates, lanolin derivatives, in an amount of for example 0.01 to 5%, preferably 0.1 to 4% by weight; physiologically compatible silicone derivatives, such as volatile or non-volatile silicone oils or high molecular weight siloxane polymers in an amount of 0.05 to 20% by weight; light protective agents, antioxidants, radical-trapping agents, antidandruff agents; fatty alcohols; vitamins; luster-imparting substances and combability-improving substances in an amount of 0.01 to 2% by weight.

### METHOD OF MAKING

The fluid composition as well as the waxy carrier can be made by conventional formulation and mixing techniques generally known to a person skilled in the art. For example the waxy carrier can be made by melting the wax ingredients together and mixing with the other optional ingredients except volatile ingredients. Subsequently the mixture is cooled. Volatile compounds are added and mixed shortly before the mixture solidifies. The waxy carrier is preferably used as a powder which can be produced by conventional milling techniques.

The non-fluid hair treatment product according to the invention which is a combination of a fluid composition absorbed on a waxy carrier, can be made by pulverizing a fluid hair styling composition (A) on a non-fluid, waxy carrier (B), wherein said fluid hair styling composition (A) contains at least one hair fixing agent dissolved or dispersed in at least one solvent which is liquid at room temperature (25 °C), and wherein said waxy carrier (B) is solid at room temperature (25 °C) and comprises at least one waxy substance. Preferred hair fixing agents are hair fixing polymers. The term "pulverizing a fluid composition on a non-fluid carrier" as used herein, means a process of making a non-fluid (e.g. powdery) end product from a fluid (e.g. liquid or gel) composition and a non-fluid (e.g. solid) carrier. The fluid is absorbed on the carrier. The term "absorbed" as used herein, means that either the surface of a non-fluid (e.g. solid) carrier particle is partly or completely coated or covered by the fluid or that the fluid is contained in cavities or pores of the carrier particle.

A general description of a method for producing a powder product from a liquid substance and a solid, powdery carrier by using compressed gases is described in WO 99/17868. The products according to the invention can be made by this method using liquid or gel-form styling compositions as the liquid substance and waxes or wax compositions as solid carriers. This method is also known as CPF-technology (Concentrated Powder Form) or as cryogenic high-pressure spray technology. In one embodiment of the invention the non-fluid hair treatment product is a product made by first dissolving a gas in a fluid hair styling composition at high pressure, then expanding the liquid/gas solution, wherein a waxy carrier is added in solid form either before, or during or shortly after said expansion. This process for producing a powdery product from a composition that is fluid at room temperature, has the steps:
- providing, in a pressure vessel, the fluid composition to be pulverized,
- dissolving a gas (e.g. carbon dioxide) in the fluid composition under elevated pressure (e.g. 100 to 250 bar),
- conducting the fluid/gas solution out of the pressure vessel to an expansion element, and
- passing the fluid/gas solution through the expansion element for rapid expansion of the solution,
wherein a waxy, powdery carrier is admixed to the fluid upstream of the expansion element, in the expansion element or downstream, in particular just downstream, of the expansion element. The obtained non-solid product can be separated from gas and remaining liquids by conventional methods, e.g. sedimentation, filtration, cyclone or electrical field. The expansion process taking place during passage of the liquid/gas solution through the expansion element can be carried out in such a manner that the temperature roughly attains or falls below the solidification temperature of the fluid composition. The gas can be dissolved in the fluid composition until the fluid composition is essentially saturated with the gas. Suitable gases are e.g. carbon dioxide, hydrocarbons, in particular methane, ethane, propane, butane, ethene, propene, or a halogenated hydrocarbon, an ether, an inert gas, in particular nitrogen, helium or argon, a gaseous oxide, in particular dinitrogen oxide or sulphur dioxide, ammonia, or a mixture of two or more of the above-mentioned gases. Most preferred is carbon dioxide. The elevated pressure under which the gas is dissolved in the fluid composition can be in the range from 5 bar to 800 bar, preferably in the range from 10 bar to 350 bar, and particularly preferably in the range from 20 bar to 250 bar. The gas can be mixed with the fluid composition, e.g. by a static mixer, by shaking or rolling the pressure vessel, by stirring the solution forming in the pressure vessel, by recirculating the liquid phase and/or gas phase present in the pressure vessel, or by a combination of two or more of the above mentioned procedures. The particle size of the powdery carrier can be less than 100 µm, preferably less than 50 µm. The amount of waxy carrier, based on the total amount of fluid composition and waxy carrier, can be between 1% by weight and 90% by weight, preferably between 10% by weight and 80% by weight, and particularly preferably between 20% by weight and 50% by weight. The expansion element can be a nozzle, a diffuser, a capillary, an orifice plate, a valve or a combination of the said expansion elements. The waxy carrier can be fed to the mass stream, which is exiting from the expansion element, in the area of the outlet point. The fluid/gas solution can be expanded into a spray tower. Gas can be additionally fed into the fluid/gas solution between the pressure vessel and the expansion element, in particular just upstream of the expansion point. The fluid/gas solution and additionally supplied gas can be expanded together with one another in the expansion element by means of a two-component nozzle. Additional gas can also be fed together with the feed of the waxy powdery carrier to the fluid composition. More details of the process are described in WO 99/17868.

### METHOD OF USE

An embodiment of the invention is a method of hair styling, said method comprising the steps of:
a) providing a non-fluid hair treatment product according to the invention described above,
b) setting the hair into a desired hair style, and
c) applying said non-fluid hair treatment product to the hair before or during setting the hair into the desired hair style without subsequent rinsing.

Such method generally involves application of an effective amount of the product to dry, slightly damp, or wet hair preferably before the hair is arranged to a desired style. The composition is then dried or allowed to dry. By "effective amount" is meant an amount sufficient to provide the hair texture, hair shine and style benefits desired considering the length and texture of the hair. In general, from about 0.5 g to about 50 g of product will be applied to the hair, depending upon the particular product formulation, length of hair, and type of hair style.

Products according to the invention of the type of the exemplary compositions described below will have benefits over conventional hair styling wax products, as well as over conventional hair styling lotions or gels in one or more of more agreeable texture and feeling to the touch; ease of distribution in hand; ease of working into hair; definition of hair; texture of hair; shine of hair; relative low overloading of the hair; no or little visible residues on hair; reduced crumbling of the product mass; good formability and good hold of the hair style. A special benefit is the unique texture and feel of the products, resembling snow flakes melting when rubbed in the hands. The products are expected to provide more shine than conventional styling lotions or styling gels and to provide more hold and are better distributable on hair than conventional styling wax products.

### EXAMPLES

The compositions illustrated in the following examples illustrate specific embodiments of the non-fluid hair treatment products of the present invention, but are not intended to be limiting thereof. Other modifications can be undertaken by the skilled artisan without departing from the spirit and scope of this invention.

The fluid styling composition and the waxy carrier compositions illustrated in the following examples are prepared by conventional formulation and mixing methods. The final products comprising the liquid styling composition absorbed on the waxy carrier is prepared by the method described in WO 99/17868 using carbon dioxide as gas for dissolving and subsequent expanding. All exemplified amounts are listed as weight percents and exclude minor materials such as diluents, preservatives, colour solutions, imagery ingredients, botanicals, and so forth, unless otherwise specified. If a trade name is mentioned as ingredient and the respective product is itself a mixture (e.g. a solution, emulsion, dispersion etc.), then the exemplified amount relates to this mixture, unless otherwise specified.

### Example 1

**Composition 1A: Fluid styling gel**

| | |
|---|---|
| 1,5 | Carbomer |
| 1,5 | Vinylacetate/crotonate copolymer (Luviset® CA 66) |
| 1,6 | Aminomethyl propanol |
| 4,2 | Sorbitol |
| 0,3 | Panthenol |
| 0,18 | PEG-40 hydrogenated castor oil |
| 0,1 | PEG-25 PABA |
| q.s. | Fragrance, preservative |
| Ad 100 | Water |

**Composition 1B: waxy carrier**

| | |
|---|---|
| 46 | PEG-60 |
| 26,3 | PEG-20 |
| 26,3 | PEG-12 |
| 0,3 | PEG-25 hydrogenated castor oil |
| 0,1 | Betaine |
| 0,1 | Glycerin (86%) |
| 0,1 | Ozokerite |
| q.s. | Fragrance |
| Ad 100 | Water |

Melting point (drop point) of carrier: 53,6 °C

Waxy composition 1B is milled into a powdery product. Gel composition 1A is absorbed on the powdery composition 1B according to the method described in WO 99/17868 using carbon dioxide as gas in an amount ratio of 5 wt% gel composition 1A to 95 wt.% waxy carrier 1B.
Melting point (drop point) of final product: 52,5 °C.

The application technical properties have been assessed by hair dresser professionals:
- the product is good distributable in the hands
- when touched or when treated on hair with a hair drier, the product melts and gets liquid
- the product gives a volume effect to hair
- when applied to the hair line, the product gives stability to the hair

### Example 2

Waxy composition 1B is milled into a powdery product. Gel composition 1A is absorbed on the powdery composition 1B according to the method described in WO 99/17868 using carbon dioxide as gas in an amount ratio of 10 wt% gel composition 1A to 90 wt.% waxy carrier 1B.
Melting point (drop point) of final product: 49,9 °C.

The application technical properties have been assessed by hair dresser professionals:
- the product is good distributable in the hands
- the product has a unique texture, which is outstanding when compared to conventional hair wax products
- the product gives stability to the hair and facilitates the forming of the desired hair style.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm".

## Claims

1. A non-fluid hair treatment product comprising a fluid composition which is absorbed on a waxy carrier, wherein the fluid composition contains at least one hair fixing agent.

2. A non-fluid hair treatment product according to claim 1 made by pulverizing a fluid hair styling composition (A) on a non-fluid, waxy carrier (B), wherein said fluid hair styling composition (A) contains at least one hair fixing polymer dissolved or dispersed in at least one solvent which is liquid at room temperature (25 °C), and wherein said waxy carrier (B) is solid at room temperature (25 °C) and comprises at least one waxy substance.

3. A non-fluid hair treatment product according to any preceding claim, wherein the product is made by first dissolving a gas in said fluid hair styling composition at high pressure, then expanding the liquid/gas solution, wherein said waxy carrier is added in solid form either before, or during or shortly after said expansion.

4. A non-fluid hair treatment product according to any preceding claim, wherein the fluid hair styling composition is a thickened lotion or a gel and contains at least one compound selected from the group consisting of gel forming agents and thickening agents.

5. A non-fluid hair treatment product according to claim 4 wherein the gel forming or thickening agent is a gel forming or thickening polymer selected from the group consisting of copolymers from at least one first type of monomer, which is selected from acrylic acid and methacrylic acid and at least one second type of monomer, which is selected from esters of acrylic acid and ethoxylated fatty alcohol; crosslinked polyacrylic acid; crosslinked copolymers from at least one first type of monomer, which is selected from acrylic acid and methacrylic acid and at least one second type of monomer, which is selected from esters of acrylic acid with C10 to C30 alcohols; copolymer from at least one first type of monomer, which is selected from acrylic acid and methacrylic acid and at least one second type of monomer, which is selected from esters of itaconic acid and ethoxylated fatty alcohol; copolymers from at least one first type of monomer, which is selected from acrylic acid and methacrylic acid, at least one second type of monomer, which is selected from esters of itaconic acid and ethoxylated C10 to C30 alcohol and a third type of monomer, selected from C1 to C4 aminoalkyl acrylates; copolymers from two or more monomers, selected from acrylic acid, methacrylic acid, acrylic acid esters, and methacrylic acid esters; copolymers from vinyl pyrrolidone and ammonium acryloyl dimethyltaurate; copolymers from ammonium acryloyl dimethyltaurate and monomers selected from esters of methacrylic acid and ethoxylated fatty alcohols; hydroxyethyl cellulose; hydroxypropyl cellulose; hydroxypropyl guar; glyceryl polyacrylate; glyceryl polymethacrylate; copolymers from at least one C2, C3, or C4 alkylene and styrene; polyurethanes; hydroxypropyl starch phosphate; polyacrylamide; copolymer crosslinked with decadiene from maleic acid anhydride and methyl vinyl ether; locust bean gum; guar gum; xanthan; dehydroxanthan; carrageenan; karaya gum; hydrolyzed corn starch; copolymers from polyethylene oxide, fatty alcohols, and saturated methylene diphenyl diisocyanate.

6. A non-fluid hair treatment product according to any preceding claim, wherein the hair fixing agent is a hair fixing polymer which is selected from the group consisting of
• anionic polymers such as terpolymers from acrylic acid, ethyl acrylate, and N-tert-butylacrylamide; crosslinked or uncrosslinked vinyl acetate/crotonic acid copolymers; terpolymers from tert.-butylacrylate, ethyl acrylate, and methacrylic acid; sodium polystyrene sulfonate; copolymers from vinyl acetate, crotonic acid, and vinyl propionate; copolymers from vinyl acetate, crotonic acid, and vinyl neodecanoate; aminomethyl propanol acrylate copolymers; copolymers from vinyl pyrrolidone and at least one additional monomer selected from acrylic acid, methacrylic acid, acrylic acid esters, and methacrylic acid esters; copolymers from methyl vinyl ether and maleic acid monoalkyl esters; aminomethyl propanol salts of copolymers from allylmethacrylate and at least one additional monomer selected from acrylic acid, methacrylic acid, acrylic acid esters, and methacrylic acid esters; crosslinked copolymers from ethyl acrylate and methacrylic acid; copolymers from vinyl acetate, mono-n-butyl maleate, and isobornyl acrylate; copolymers from two or more monomers selected from acrylic acid, methacrylic acid, acrylic acid esters, and methacrylic acid esters, copolymers from octylacrylamide and at least one monomer selected from acrylic acid, methacrylic acid, acrylic acid esters, and methacrylic acid esters; and polyesters from diglycol, cyclohexanedimethanol, isophthalic acid, and sulfoisophthalic acid,
• cationic polymers such as cationic cellulose derivatives from hydroxyethyl cellulose and diallyl dimethyl ammonium chloride; cationic cellulose derivatives from hydroxyethyl cellulose and epoxide substituted with trimethyl ammonium; poly-(dimethyldiallyl ammonium chloride); copolymers from acrylamide and dimethyldiallyl ammonium chloride; quaternary ammonium polymers, formed by the reaction of diethylsulfate and a copolymer from vinyl pyrrolidone and dimethylaminoethyl methacrylate; quaternary ammonium polymers from methylvinylimidazolium chloride and vinyl pyrrolidone; Polyquaternium-35; polymer from trimethyl ammonium ethyl methacrylate chloride; Polyquaternium-57; dimethylpolysiloxane terminally substituted with quaternary ammonium groups; copolymer from vinyl pyrrolidone, dimethylaminopropyl methacrylamide, and methacryloylamino propyl lauryl dimethyl ammonium chloride; chitosan and salts thereof; hydroxyalkyl chitosans and salts thereof; alkyl hydroxyalkyl chitosans and salts thereof; N-hydroxyalkyl chitosan alkyl ether; copolymer from vinyl caprolactam, vinyl pyrrolidone, and dimethylaminoethyl methacrylate; copolymers from vinyl pyrrolidone and dimethylaminoethyl methacrylate, copolymers from vinyl pyrrolidone, vinyl caprolactam, and dimethylaminopropylacrylamide; poly- or oligo-esters, constructed from at least one first type of monomer, which is selected from hydroxycarboxylic acid substituted with at least one quaternary ammonium group,
• amphoteric or zwitterionic polymers such as copolymers from octylacrylamide, acrylic acid, butylaminoethyl methacrylate, methyl methacrylate, and hydroxypropyl methacrylate; copolymers from lauryl acrylate, stearyl acrylate, ethylamine oxide methacrylate, and at least one monomer selected from acrylic acid, methacrylic acid, acrylic acid esters, and methacrylic acid esters; copolymers from methacryloyl ethyl betaine and at least one monomer selected from methacrylic acid and methacrylic acid esters; copolymers from acrylic acid, methylacrylate, and methacrylamide propyl trimethylammonium chloride; oligomers or polymers that can be produced from quaternary crotonic betaines or quaternary crotonic betaine esters,
• nonionic polymers such as polyvinylpyrrolidone, polyvinyl caprolactam, vinyl pyrrolidone/vinylacetate copolymers, polyvinylalcohol, isobutylene/ethylmaleimide/hydroxyethylmaleimide copolymer; copolymers from vinyl pyrrolidone, vinyl acetate, and vinyl propionate.

7. A non-fluid hair treatment product according to any preceding claim, wherein the waxy carrier comprises at least one waxy substance selected from the group consisting of paraffin waxes, polyolefin waxes, wool wax, wool wax alcohols, candelilla wax, olive wax, carnauba wax, Japan wax, apple wax, hydrogenated fats, fatty acid esters, fatty acid glycerides, fatty acid triglycerides, fatty acids, fatty alcohols, polyethylene glycol waxes, and silicone waxes.

8. A non-fluid hair treatment product according to any preceding claim, wherein the fluid composition is a hair styling gel composition having a viscosity of at least 250 mPa s and containing in an aqueous or aqueous-ethanolic solvent
(A) 0.5 to 10 wt.% based on the gel composition of at least one gel forming polymer; and
(B) 0.1 to 15 wt.% based on the gel composition of at least one dissolved or dispersed hair fixing polymer, selected from the group consisting of anionic hair fixing polymers and nonionic hair fixing polymers.

9. Method of hair styling, wherein
- a non-fluid hair treatment product according to any preceding claim is provided,
- the hair is set into a desired hair style, and
- said hair treatment product is applied to the hair before or during setting the hair into the desired hair style, without subsequent rinsing.

10. Use of a product according to any of claims 1 to 8 for styling human hair.
